# EUROPÄISCHE PATENTSCHRIFT

(11) **EP 1 568 384 B1**
(45) Veröffentlichungstag und Bekanntmachung des Hinweises auf die Patenterteilung: **05.09.2007**
(21) Anmeldenummer: 04405091.2
(22) Anmeldetag: 18.02.2004
(51) Int. Cl.: A61L 2/16, A61L 2/24, D21C 5/02, D21H 21/04

(54) **Verfahren zur Stabilisierung von verkeimbaren flüssigen Medien**
Process for stabilizing germinative fluid media
Procédé de stabilisation des médias fluides germinatifs

(43) Veröffentlichungstag der Anmeldung: 31.08.2005
(73) Patentinhaber: Sappi Austria Produktions-GmbH & Co. KG., 8101 Gratkorn (AT)
(72) Erfinder: ZACH Raimund, 8114 Stübing (AT)
(74) Vertreter: Bremi, Tobias Hans

(56) Entgegenhaltungen:
- EP-A- 0 447 672
- EP-A- 0 524 917
- EP-A- 0 602 391
- EP-A- 0 688 500
- DE-A- 19 850 170
- US-A- 3 652 383

## Beschreibung

### TECHNISCHES GEBIET

Die vorliegende Erfindung betrifft ein Verfahren zur Stabilisierung von im wesentlichen flüssigen Medien wie z.B. Suspensionen, die, in einem Behälter gelagert, aeroben und/oder anaeroben Abbauprozessen unterworfen sind. Insbesondere betrifft sie die Stabilisierung von flüssigen oder suspendierten Medienstoffen, welche in einer Papierfabrik eingesetzt werden.

### STAND DER TECHNIK

Beispielsweise im Bereich der Papierherstellung müssen immer wieder Behälter mit Ausschusszellstoff über längere Zeit stabilisiert werden, um zu verhindern, dass durch eine zu starke Verkeimung der Behälterinhalt unbrauchbar wird. Unter einer Verkeimung ist in diesem Zusammenhang eine bakterielle, anaerobe oder aerobe Stoffwechselreaktion des Behälterinhalts zu verstehen. Diese Vorgänge laufen ohne entsprechende Kontrolle in derartigen Medien unvermeidbar aufgrund der nährstoffreichen, meist zusätzlich auf der idealen Temperatur befindlichen Umgebung ab.

Nimmt die Verkeimung ein gewisses Ausmass an, so wird der Inhalt eines derartigen Behälters unbrauchbar. Begleitet wird eine starke aerobe / anaerobe Verkeimung bereits im Anfangsstadium durch eine unerwünschte Geruchsbildung im Behälter.

Unter "unbrauchbar" versteht man dabei, dass der Einsatz des verkeimten Lagerbehälterinhaltes zu hohen Folgekosten im Prozess oder inakzeptablen Prozessstörungen z.B. bei der Papierherstellung führt. Typischerweise wird entsprechend vorbeugend normalerweise dem Medium bei stetem Rühren diskontinuierlich ein Biozid zugeführt, welches die aerobe / anaerobe Verkeimung verhindern soll.

Derartige Verfahren können dabei auch vorbeugend angewandt werden, um ein "Kippen" des Behälterinhaltes in einen unbrauchbaren Zustand von vornherein auszuschliessen.

Da es sich bei den Bioziden um ökologisch nicht unproblematische Substanzen handelt (vgl. dazu beispielsweise die europäische Richtlinie 98/8/EC), ist ein haushälterischer Umgang mit derartigen Chemikalien heutzutage vorgeschrieben, und die Stabilisierung ist beispielsweise nicht einfach durch eine übermässige Zugabe von Bioziden realisierbar.

### DARSTELLUNG DER ERFINDUNG

Der Erfindung liegt demnach die Aufgabe zugrunde, ein verbessertes respektive alternatives Verfahren zur Stabilisierung und/oder Entkeimung von im wesentlichen flüssigen, in wenigstens einem Behälter gelagerten, aeroben und/oder anaeroben Abbauprozessen unterworfenen Medien wie z.B. Suspensionen vorzuschlagen.

Im wesentlichen geht es dabei darum, die Lagerzeit zu verlängern, und/oder die Biozidmengen zu reduzieren, und damit ein ökologisch verbessertes Verfahren zu erreichen.

Die Lösung dieser Aufgabe wird dadurch erreicht, dass wenigstens zeitweise dem Medium Wirkstoffe und/oder Zubereitungen, die einen oder mehrere Wirkstoffe enthalten, die dazu bestimmt sind, auf insbesondere chemischem Wegeein zu rasches Wachsen von Schadorganismen zu verhindern, zugeführt werden, und dass gleichzeitig dem Medium Luft zudosiert wird.

Der Kern der Erfindung besteht somit darin, zu erkennen, dass eine Kombination der Zugabe von Biozid und Luft eine verlängerte Lagerungsfähigkeit von derartigen Medien erlaubt.

Typischerweise können beispielsweise im Bereich der Lagerung von Pulpen aus der Papierherstellung Lagerungszeiten erreicht werden, welche dreimal so gross sind, wie Lagerungszeiten bei konventioneller Behandlung. Während also typischerweise derartige Behälter bei Verfahren nach dem Stand der Technik nach 2-3 Tagen Lagerung unbrauchbar sind, können Sie bei Anwendung des vorgeschlagenen Verfahrens über eine Woche gehalten werden.

Die kombinierte Zugabe von sowohl Luft als auch Biozid erfolgt dabei vorzugsweise in Intervallen (schubweise), normalerweise zusammen mit einer Umwälzung des Behälterinhalts.

Gemäss einer ersten bevorzugten Ausführungsform des Verfahrens wird die Luft im Bodenbereich und/oder im bodennahen Bereich des Behälters zudosiert. Insbesondere bei einer derartigen Zuführung können die anaeroben Prozesse im Behälter durch die Luftzugabe optimal beeinflusst werden. Die Luftzugabe kann dabei durch spezifische im Bodenbereich angeordnete Leitungen in verteilter Weise geschehen.

In einem konkreten Anwendungsfall wird in einem 2000 m³ Turm 200 Nm³/h Luft in einem Intervall über einen Zeitraum von 2 Std. kombiniert mit einem speziellen Biozid zugesetzt. Es erweist sich somit als vorteilhaft, pro 5 m³ Medium im Bereich von 0.3-1.5 m³, bevorzugt im Bereich von 0.5m³ Luft zuzudosieren. Dies normalerweise bei einem Feststoffgehalt im Medium von ca. 30-60 g/l.

Es zeigt sich, dass es in den meisten Fällen genügend ist, Umgebungsluft einzubringen, welche insbesondere bevorzugt über einen Kompressor eingeblasen wird.

Die für einen derartigen Einsatz in Kombination mit Luft verwendbaren Biozide können von der Firma Biomontan (AT) unter dem Handelsnamen "Petrosid" erhalten werden. Es handelt sich mit anderen Worten vorzugsweise um ein Biozid und/oder eine biozide Zubereitung (wenigstens teilweise enthaltend biozide Wirkstoffe), welche auf chemischem Wege die anaerobe und/oder insbesondere die aerobe Verkeimung von bevorzugt biologischen wässrigen organischen Systemen verhindert respektive rückgängig macht. Weiterhin bevorzugt enthält eine derartige Zusammensetzung zudem Stabilisatoren.

Beim flüssigen Medium, welches stabilisiert werden soll, handelt es sich beispielsweise um eine wässrige Lösung, Suspension und/oder eine wässrige Aufschlämmung von organischem biologischem Material, wobei es sich bevorzugt beim organischen biologischen Material um Eiweisse, Kohlenwasserstoffe, Stärke, Zellulose, Glykogene oder eine Mischung davon handelt. Aber auch alle anderen flüssigen Medien, welche biologischen Abbauprozessen unterworfen sind, können mit diesem Verfahren stabilisiert werden. Insbesondere z.B. handelt es sich beim flüssigen Medium im wesentlichen um eine wässrige Aufschlämmung von Zellulose und Stärke, insbesondere um eine Zellstoff-Aufschlämmung aus der Papierherstellung.

Die Zugabe von Luft und Biozid wird in Abhängigkeit eines im Medium gemessenen pH- und/oder Redox-Wertes und/oder in Abhängigkeit der Keimzahl im Medium vorgenommen. So lässt sich der Prozess optimal steuern, und insbesondere die Zugabe von Biozid auf dem Minimum des erforderlichen einstellen. Insbesondere erweist es sich im Bereich der Papierverarbeitung als vorteilhaft, den Wirkstoff und/oder die Zubereitung in einer Menge im Bereich von 10 bis 50 ppm, bevorzugt ca. 25 ppm (Volumenanteile Biozid bezogen auf den Behälterinhalt *atro*, d.h. absolut trocken) zuzudosieren.

Typischerweise sollte dabei zur Erhöhung der Lagerungszeiten der Wirkstoff und/oder die Zubereitung in Abhängigkeit der im Medium vorhandenen Bakterienkultur ausgewählt werden, respektive sukzessive dem zeitlichen Verlauf dieser Bakterienkultur angepasst werden.

Das Verfahren lässt sich insbesondere dann vorteilhaft anwenden, wenn der Behälter wenigstens eine Rezirkulationsleitung zur Umwälzung des Mediums aufweist, mit welcher Medium im Bodenbereich und/oder im bodennahen Bereich eingebracht wird, wobei die Luft dann z.B. in wenigstens eine dieser Rezirkulationsleitungen eingeblasen wird. Alternativ kann Luft auch über Düsen oder über Luftlanzen in den Behälter eingebracht werden. Über dieselbe Rezirkulationsleitung kann auch das Biozid zugeführt werden. Das Biozid kann aber auch über andere Kanäle zugeführt werden. Es sollte dabei möglichst homogen im Behälter verteilt werden. Da der Behälter typischerweise über ein Rührwerk verfügt, kann die Verteilung unter anderem über dieses Rührwerk gewährleistet werden.

Wie bereits weiter oben erwähnt, kann das vorgeschlagene Verfahren besonders effizient für die Lagerung von flüssigen resp. suspendierten Medien eingesetzt werden, welcher in einer Papierfabrik zwischengelagert werden soll.

Weitere bevorzugte Ausführungsformen des erfindungsgemässen Verfahrens sind in den abhängigen Ansprüchen beschrieben.

Weiterhin betrifft die vorliegende Erfindung einen Wirkstoff und/oder Zubereitung, die einen oder mehrere Wirkstoffe enthalten, die dazu bestimmt sind, auf chemischem und/oder biologischem Wege Schadorganismen zu zerstören, abzuschrecken, unschädlich zu machen, Schädigungen durch sie zu verhindern oder sie in anderer Weise zu bekämpfen zur Verwendung in einem Verfahren, wie es oben beschrieben ist.

Weitere bevorzugte Ausführungsformen sind in den abhängigen Ansprüchen beschrieben.

### WEGE ZUR AUSFÜHRUNG DER ERFINDUNG

Das vorgeschlagene Luft-Biozid-Verfahren dient dazu, flüssige Medien in Lagerbehältern, welche durch aerobe / anaerobe Verkeimung unbrauchbar geworden sind, zu retten bzw. in einen brauchbaren Zustand zurückzuführen, respektive derartige flüssige Medien in Lagerbehältern vor einer entsprechenden Verkeimung zu bewahren (Prävention).

Konkret soll im vorliegenden Fall als Ausführungsbeispiel ein Behälter mit Ausschusszellstoff aus der Papierherstellung betrachtet werden, es muss aber betont werden, dass das vorliegende Verfahren grundsätzlich auf alle flüssigen Medien, d. h. wässrigen Lösungen oder Suspensionen aus Substanzen respektive Festkörperbestandteilen anwendbar ist, welche durch aerobe/anaerobe Verkeimung unbrauchbar werden können. So kommen beispielsweise gleichermassen in Frage: Lagerbehälter für Rückwasser, Deinkstoff, Zellstoff, Fruchtsäfte, Öle, Slurries.

Unter "unbrauchbar" versteht man in diesem Zusammenhang, dass der Einsatz des verkeimten Lagerbehälterinhaltes zu hohen Folgekosten im Prozess oder inakzeptablen Prozessstörungen führt. So beispielsweise, wenn es sich beim flüssigen Medium um Ausschusszellstoff aus der Papierherstellung handelt, da im anschliessenden Herstellungsprozess von Papier die Anwesenheit von Verkeimung zu Prozessschwierigkeiten und ähnlichem Anlass geben kann.

Begleitet wird die aerobe / anaerobe Verkeimung bereits im Anfangsstadium normalerweise durch eine unerwünschte Geruchsbildung im Behälter. Durch Anwendung des Verfahrens kann diese Geruchsbildung verhindert werden, der pH-Wert angehoben und der Redox-Wert Richtung positiv verschoben werden.

Das Verfahren kann auch vorbeugend angewandt werden, um ein "Kippen" des Behälterinhaltes in einen unbrauchbaren Zustand von vornherein auszuschliessen. Zusätzlich können damit Biozidbehandlungskosten gesenkt werden, die Wirkung von Bioziden erhöht werden oder Prozesschemikalien eingespart werden.

### Allgemeine Beschreibung des Verfahrens:

Das Luft-Biozid-Verfahren ist insbesondere anwendbar, wenn im Behälter aerobe sowie auch anaerobe Bedingungen vorliegen oder der Behälter droht, vom aeroben Bereich in den anaeroben Bereich zu "kippen".

Meist beginnt die Verkeimung des Behälterinhaltes unter aeroben Bedingungen. Diese überwiegen, solange genügend Sauerstoff vorhanden ist.

Der Wechsel in den anaeroben Bereich kann durch eine online pH- und / oder Redoxmessung am Stoffaustrag oder durch eine entsprechende Labormessung (ggf. auch online) der Verkeimung nachgewiesen werden. Kippt der Behälter z.B. bei der Papierverarbeitung in den sauren pH Bereich und/oder wird ein Redox-Potenzial unterhalb von ca. -100 mV gemessen, so kann davon ausgegangen werden, dass der Wechsel in den anaeroben Bereich stattgefunden hat.

Bei Unterschreitung derartiger Grenzwerte bei Redox und pH bzw. Überschreitung einer bestimmten Anzahl von anaeroben Keimen im Behälter wird Luft im Bodenbereich des Behälters dosiert. Die Luft wird dabei zugeführt, indem Aussenluft über einen Kompressor im Bodenbereich eingeblasen wird. Typischerweise reicht es dabei z.B. im Bereich der Papierverarbeitung, pro 5 m³ Behälterinhalt ca. 0.5m³ Luft einzublasen.

Bevorzugt gleichzeitig mit der Luft wird erfindungsgemäss nun Biozid in den Behälterinhalt dosiert. Das Biozid wird dabei der Bakterienkultur des Behälterinhaltes angepasst. Es kann ausserdem Konservierungsmittel enthalten.

Wenn im Behälter keine ausreichende Durchmischung durch ein Rührwerk gewährleistet ist, um das Biozid einzurühren, so kann dieses über eine Rezirkulationsleitung, welche dazu vorgesehen ist, den Behälterinhalt umzuwälzen, dosiert werden.

Die Messungen von pH oder Redox oder Keimzahl sind vorteilhaft, wenn eine vorbeugende Behandlung des Behälterinhaltes erfolgen soll. Sie sind auch vorteilhaft, um die Wirksamkeit der Behandlung eines unbrauchbaren Behälterinhaltes mit dem beschriebenen Verfahren kontrolliert durchführen zu können, und um zu gewährleisten, dass Biozid in ökologisch optimalem Masse eingesetzt wird.

Im Resultat zeigt es sich, dass im Bereich derartiger Behälter mit Ausschusszellstoff in der Papierindustrie Lagerungszeiten von über 7 Tage sogar bis 10 Tage möglich sind, während normalerweise derartige Behälter unter ausschliesslicher Verwendung von Biozid nur 2-3 Tage gelagert werden können, anschliessend ist die Verkeimung derart fortgeschritten, dass der Behälterinhalts nicht mehr in den Prozess geführt werden kann.

Die Zuführung von Luft wie auch die Zuführung von Biozid kann in Intervallen von z.B. ca. 2h vorgenommen werden, in welchen beide Komponenten zugeführt werden.

### Beispiel:

Ein Lagerturm für gestrichenen Ausschuss an einer Papiermaschine zur Herstellung von holzfreien gestrichenen Papieren (Ausschussturm) mit einem Inhalt von 2000 m³ soll in Bezug auf Verkeimung/pH/Redoxwert stabilisiert werden.

Das Ziel ist die Stabilisierung der Verkeimung, und entsprechend des pH-Wertes und des Redoxwertes in diesem Turm. Der Festkörperanteil im Turm beträgt dabei 30-60 g/l, d.h. 3-6 Gew.-%.

PH und Redox werden in einer Rezirkulationsleitung gemessen unter Verwendung von Standard-Elektrodenmethoden. Unter " guten " Bedingungen wird dabei ein pH Wert im Bereich von 8 gemessen, und ein Redox-Potenzial von ca. +100 mV. Die Rezirkulationsleitung wird mit einer separaten Pumpe betrieben, wobei der Behälterinhalt innerhalb von 2 - 8h vollständig um umgewälzt wird. Der Stoff wird am Behälterboden abgezogen und im oberen Behälterteil wieder eingebracht.

Der Inhalt weist einen sehr hohen gelösten Stärkeanteil auf (ca. 0.1 Gew.-%), d. h. für Bakterien liegt Nahrung immer im Überschuss vor. Diese Stärke ist der ideale Nährstoff für Bakterien. Daher verkeimt der Behälterinhalt innerhalb von einigen Stunden, wobei insbesondere die fakultativ anaeroben Bakterium störend sind. Dies ist messbar durch einen Abfall des pH- Wertes von 8 auf ungefähr 6 und des Redoxwertes von +100 mV auf ca. -300 mV, da die Bakterien Säuren beim Stoffwechsel ausscheiden.

Am Anfang findet vorwiegend ein Wachstum der Bakterien unter aeroben Bedingungen statt. Wenn der Sauerstoff im Wasser verbraucht ist, wechseln fakultativ aerobe Bakterien in den anaeroben Bereich. Diese Art der Bakterien ist in diesem Behälter in grosser Anzahl zu finden. Der Wechsel in den anaeroben Bereich kann durch einen Abfall des Redoxpotentiales und des pH-Wertes gemessen werden Turm auf Vergleich oben). Als Schwellenwerte kann dabei für den pH normalerweise von 7 ausgegangen werden, und für das Redox Potenzial von -200mV.

In die Rezirkulationsleitung des Behälters wird regelmässig (d.h. z.B. einmal pro Tag) aber diskontinuierlich Biozid (d.h. z.B. jeweils während 2-4 Stunden) zugegeben um den Stoff zu Konservieren und die Bakterienpopulation unter einem gewissen Maximalwert zu halten.

Wenn aber die Verweilzeit des Stoffes durch zu geringe Abnahme der nachfolgenden Papiermaschine zu hoch wird, so steigt das Niveau der Verkeimung und ein "Kippen" in den anaeroben Bereich wird beobachtet. Die pH- und Redoxmessungen in der Rezirkulationsleitung zeigen diesen Punkt an (Unterschreiten der oben genannten Schwellenwerte).

Wenn das Wachstum der anaeroben Bakterien und fakultativ anaeroben Bakterien beginnt, wird Luft in den Behälter dosiert. Die Luftdosierung erfolgt entweder über die Rezirkulationsleitung oder über einige Dosierdüsen im konischen Bodenbereich.

Die Luftdosierung beträgt 100 Nm³/h (Nm³ = Normkubikmeter) über einen Zeitraum von ca. 4 Stunden (der Zeitraum hängt unter anderem von der Intensität der Verkeimung und von der Art des Mediums ab). Ein nächster derartiger Zyklus wird erst wieder ausgelöst, wenn die Schwellenwerte erneut unterschritten werden. Die Luftdosierung innerhalb eines Intervalls wird der Entwicklung des pH- und Redoxwertes angepasst, d.h. es wird solange dosiert, bis eine zufriedenstellende Entwicklung bei pH und Redox beobachtet werden kann (d. h. die Schwellenwerte werden wieder überschritten).

Gleichzeitig mit der Luft wird Biozid beigemischt. Dies in einer Menge von ca. 25ppm (Vol. Biozid/kg atro Inhalt). Frage kommt beispielsweise das Biozid Petrosid D20, wie es von der Firma Biomontan (AT) erhältlich ist.

Zusätzlich wird die Biozidmenge und -type während der Luftdosierung angepasst. Dabei wird zuerst wird mit einer Kombination Luft und/oder Killer-Biozid die Bakterienpopulation reduziert, danach werden mit einer Kombination Luft und/oder Konservierungsmittel stabile Voraussetzungen für die nächsten 8-24 Stunden geschaffen.

Wenn das Verfahren vorbeugend praktiziert wird, so werden die Grenzwerte bei der Verweilzeit, beim pH- und Redoxwert relativ nahe an den Idealzustand gelegt. Wird das Verfahren vorbeugend praktiziert, so wird regelmässig, d. h. beispielsweise einmal pro Tag oder alle zwei Tage, während eines vorgegebenen Intervalls von beispielsweise 4 Stunden Luft eingeblasen und Biozid zugeführt. Die Länge der Intervalle, deren zeitlicher Abstand und die Menge der zugeführten Luft respektive des zugeführten Biozids wird dabei so eingestellt, dass sich die Messwerte nie zu weit unter die Schwellenwerte bewegen. Auf diese Weise wird wesentlich weniger Biozid als bei Verfahren nach dem Stand der Technik eingeführt, und es können dennoch vorbeugend stabile Verhältnisse gewährleistet werden.

## Patentansprüche

1. Verfahren zur Stabilisierung und/oder Entkeimung von im wesentlichen flüssigen, in wenigstens einem Behälter gelagerten, aeroben und/oder anaeroben Abbauprozessen unterworfenen Medien wie insbesondere Suspensionen,
**dadurch gekennzeichnet, dass**
wenigstens zeitweise dem Medium Wirkstoffe und/oder Zubereitungen, die einen oder mehrere Wirkstoffe enthalten, die dazu bestimmt sind, auf chemischem Wege Schadorganismen zu zerstören, abzuschrecken, unschädlich zu machen, Schädigungen durch sie zu verhindern oder sie in anderer Weise zu bekämpfen, kontrolliert zugeführt werden,
und dass gleichzeitig oder zeitversetzt dem Medium Luft zudosiert wird,
wobei die kombinierte Zugabe von Biozid-Wirkstoff respektive von Luft in Abhängigkeit eines im Medium gemessenen pH- und/oder Redox-Wertes und/oder in Abhängigkeit der Keimzahl im Medium vorgenommen wird und
wobei die kombinierte Zugabe von Biozid-Wirkstoff ausgelöst wird, wenn der gemessene pH-Wert in eine Bereich unterhalb pH 7 kippt und/oder wenn das Redox-Potenzial auf unterhalb -200 mV fällt.

2. Verfahren nach Anspruch 1, **dadurch gekennzeichnet, dass** die Luft im Bodenbereich und/oder im bodennahen Bereich des Behälters zudosiert wird.

3. Verfahren nach einem der vorhergehenden Ansprüche, **dadurch gekennzeichnet, dass** pro 5 m³ Medium im Bereich von 0.3-1.5 m³ (Normkubikmeter), bevorzugt im Bereich von 0.5m³ Luft zudosiert wird.

4. Verfahren nach Anspruch 3, **dadurch gekennzeichnet, dass** es sich bei der Luft um Umgebungsluft handelt, welche insbesondere bevorzugt über einen Kompressor eingeblasen wird.

5. Verfahren nach einem der vorhergehenden Ansprüche, **dadurch gekennzeichnet, dass** es sich bei den Wirkstoffen und/oder Zubereitungen um Biozide wie Schutz- und/oder Konservierungsmittel für Flüssigkeiten in Kühl- und Verfahrenssystemen insbesondere aus dem Bereich der Papierindustrie handelt.

6. Verfahren nach Anspruch 5, **dadurch gekennzeichnet, dass** es sich um ein Biozid und/oder eine biozide Zubereitung handelt, welche auf chemischem und/oder biologischem Wege die anaerobe und/oder aerobe Verkeimung von wässrigen insbesondere suspendierten Medien Systemen verhindert respektive rückgängig macht.

7. Verfahren nach einem der vorhergehenden Ansprüche, **dadurch gekennzeichnet, dass** es sich beim flüssigen Medium um eine wässrige Lösung resp. eine Suspension von anorganischen und/oder organischen Stoffen handelt, wobei es sich bevorzugt bei den Stoffen um Eiweisse, Kohlehydrate und/oder andere Kohlenwasserstoffe resp. organische Systeme oder eine Mischung davon handelt.

8. Verfahren nach Anspruch 7, **dadurch gekennzeichnet, dass** es sich beim flüssigen Medium im wesentlichen um eine wässrige Aufschlämmung von Zellulose und Stärke aus der Papierherstellung handelt.

9. Verfahren nach Anspruch 8, **dadurch gekennzeichnet, dass** es sich beim flüssigen Medium um eine Zellstoff Aufschlämmung aus der Papierherstellung mit einem Feststoffanteil im Bereich von 0.1-80 g/l, bevorzugt von 30 bis 60 g/l handelt.

10. Verfahren nach einem der vorhergehenden Ansprüche, **dadurch gekennzeichnet, dass** der Wirkstoff und/oder die Zubereitung in einer Menge von 10 bis 100 ppm, bevorzugt ca. 25 bis 50 ppm zudosiert werden.

11. Verfahren nach einem der vorhergehenden Ansprüche, **dadurch gekennzeichnet, dass** der Wirkstoff und/oder die Zubereitung in Abhängigkeit der im Medium vorhandenen Bakterienkultur ausgewählt ist, respektive dem zeitlichen Verlauf dieser Bakterienkultur angepasst wird.

12. Verfahren nach einem der vorhergehenden Ansprüche, **dadurch gekennzeichnet, dass** der Behälter wenigstens eine Rezirkulationsleitung zur Umwälzung des Mediums aufweist, mit welcher Medium im Bodenbereich und/oder im bodennahen Bereich eingebracht wird, und dass die Luft bevorzugt in wenigstens eine dieser Rezirkulationsleitungen eingeblasen wird.

13. Verfahren nach einem der vorhergehenden Ansprüche, **dadurch gekennzeichnet, dass** es sich beim Medium um Ausschusszellstoff handelt, welcher in einer Papierfabrik zwischengelagert werden soll.

## Claims

1. Process for stabilizing and/or sterilizing substantially fluid media stored in at least one container and subjected to aerobic and/or anaerobic degradation processes, such as, in particular, suspensions, **characterized in that** active substances and/or preparations which contain one or more active substances which are intended for destroying, quenching or rendering harmless harmful organisms by a chemical method, preventing damage by them or controlling them in another manner are fed at least temporarily to the medium in a controlled manner, and **in that** at the same time or at a different time air is metered into the medium, the combined addition of biocidal active substance and of air being carried out as a function of a pH and/or redox value measured in the medium and/or as a function of the microbial count in the medium and the combined addition of biocidal active substance being triggered when the measured pH flips into a range below pH 7 and/or when the redox potential falls below -200 mV.

2. Process according to Claim 1, **characterized in that** the air is metered in in the bottom region and/or **in that** region of the container which is close to the bottom.

3. Process according to either of the preceding claims, **characterized in that** in the range of 0.3-1.5 m³ (cubic metres at standard temperature and pressure), preferably in the region of 0.5 m³, of air is metered in per 5 m³ of medium.

4. Process according to Claim 3, **characterized in that** the air is ambient air which is particularly preferably blown in via a compressor.

5. Process according to any of the preceding claims, **characterized in that** the active substances and/or preparations are biocides, such as protective agents and/or preservatives for fluids in cooling and process systems, in particular from the area of the paper industry.

6. Process according to Claim 5, **characterized in that** the biocide is a biocide and/or a biocidal preparation which prevents or eliminates the anaerobic and/or aerobic microbial contamination of aqueous, in particular suspended, media systems by a chemical and/or biological method.

7. Process according to any of the preceding claims, **characterized in that** the fluid medium is an aqueous solution or a suspension of inorganic and/or organic substances, the substances preferably being proteins, carbohydrates and/or other hydrocarbons or organic systems or a mixture thereof.

8. Process according to Claim 7, **characterized in that** the fluid medium is substantially an aqueous suspension of cellulose and starch from papermaking.

9. Process according to Claim 8, **characterized in that** the fluid medium is a pulp suspension from papermaking, having a solids content in the range of 0.1-80 g/l, preferably of 30 to 60 g/l.

10. Process according to any of the preceding claims, **characterized in that** the active substance and/or the preparation are metered in in an amount of 10 to 100 ppm, preferably about 25 to 50 ppm.

11. Process according to any of the preceding claims, **characterized in that** the active substance and/or the preparation is selected depending on the bacterial culture present in the medium or is adapted to the progress of this bacterial culture.

12. Process according to any of the preceding claims, **characterized in that** the container has at least one recirculation line for circulating the medium, by means of which medium is introduced in the bottom region and/or in the region close to the bottom, and **in that** the air is preferably blown into at least one of these recirculation lines.

13. Process according to any of the preceding claims, **characterized in that** the medium is waste pulp which is to be temporarily stored in a paper mill.

## Revendications

1. Procédé en vue de la stabilisation et/ou du dégermage de milieux pour l'essentiel fluides, stockés dans au moins un récipient, soumis à des processus de dégradation aérobies et/ou anaérobies, comme, en particulier des suspensions,
**caractérisé**
**en ce que** l'on ajoute, d'une manière contrôlée, au moins par moments, au milieu des agents actifs et/ou des préparations, qui contiennent un ou plusieurs agents actifs, qui sont destinés à détruire, à tremper, à rendre inoffensifs, par voie chimique, des organismes nocifs, à empêcher l'apparition de dégâts causés par ces derniers ou à lutter de toute autre manière contre ces derniers, et
**en ce que** l'on ajoute par dosage au milieu, simultanément ou avec un décalage dans le temps, de l'air,
l'addition combinée d'agent actif à effet biocide, respectivement d'air, étant entreprise en fonction d'une valeur de pH et/ou d'une valeur rédox mesurée dans le milieu et/ou en fonction du nombre de germes dans le milieu et
l'addition combinée d'agent actif à effet biocide étant déclenchée lorsque la valeur de pH mesurée bascule dans un domaine en dessous du pH 7 et/ou lorsque le potentiel rédox tombe en dessous de -200 mV.

2. Procédé selon la revendication 1, **caractérisé en ce que** l'air est ajouté par dosage dans le domaine de fond et/ou dans le domaine proche du fond du récipient.

3. Procédé selon l'une quelconque des revendications précédentes, **caractérisé en ce que** l'on ajoute par dosage, pour 5 m³ de milieu, dans le domaine de 0,3-1,5 m³ (mètres cubes normés), de préférence, dans le domaine de 0,5 m³ d'air.

4. Procédé selon la revendication 3, **caractérisé en ce qu'**il s'agit, dans le cas de l'air, de l'air environnant, qui est insufflé tout particulièrement, de préférence, à l'aide d'un compresseur.

5. Procédé selon l'une quelconque des revendications précédentes, **caractérisé en ce qu'**il s'agit, dans le cas des agents actifs et/ou des préparations, d'agents biocides tels que des agents de protection et/ou de conservation pour fluides dans des systèmes de réfrigération ou des systèmes de procédés, en particulier en provenance du domaine de l'industrie du papier.

6. Procédé selon la revendication 5, **caractérisé en ce qu'**il s'agit d'un agent biocide et/ou d'une préparation biocide, qui empêche, respectivement annule, par voie chimique et/ou biologique, le développement anaérobie et/ou aérobie de germes de systèmes de milieux aqueux, en particulier mis en suspension.

7. Procédé selon l'une quelconque des revendications précédentes, **caractérisé en ce qu'**il s'agit, dans le cas du milieu fluide, d'une solution aqueuse respectivement d'une suspension de substances inorganiques et/ou organiques, les substances étant, de préférence, des albumens, des glucides et/ou d'autres hydrocarbures, respectivement des systèmes organiques ou un mélange de ceux-ci.

8. Procédé selon la revendication 7, **caractérisé en ce qu'**il s'agit, dans le cas du milieu fluide, pour l'essentiel d'une suspension aqueuse de cellulose et d'amidon provenant de la fabrication du papier.

9. Procédé selon la revendication 8, **caractérisé en ce qu'**il s'agit, dans le cas du milieu fluide, d'une suspension de pâte de cellulose provenant de la fabrication du papier, ayant une proportion de matières solides dans le domaine de 0,1-80 g/l, de préférence, de 30 à 60 g/l.

10. Procédé selon l'une quelconque des revendications précédentes, **caractérisé en ce que** l'agent actif et/ou la préparation est ajouté(e) par dosage dans une quantité de 10 à 100 ppm, de préférence, d'environ 25 à 50 ppm.

11. Procédé selon l'une quelconque des revendications précédentes, **caractérisé en ce que** l'agent actif et/ou la préparation est sélectionné(e) en fonction de la culture de bactéries présente dans le milieu, respectivement en fonction de la variation dans le temps du développement de cette culture de bactéries.

12. Procédé selon l'une quelconque des revendications précédentes, **caractérisé en ce que** le récipient présente au moins un conduit de recirculation en vue de la mise en circulation du milieu, grâce auquel le milieu est introduit dans le domaine de fond et/ou dans domaine proche du fond, et **en ce que** l'air est insufflé, de préférence, dans au moins un de ces conduits de recirculation.

13. Procédé selon l'une quelconque des revendications précédentes, **caractérisé en ce qu'**il s'agit, dans le cas du milieu, d'une pâte de cellulose de rebut, qui doit être stockée à titre provisoire dans une fabrique de papier.
